# EUROPEAN PATENT SPECIFICATION

(11) **EP 2 084 544 B1**
(45) Date of publication and mention of the grant of the patent: **06.07.2011**
(21) Application number: 07823236.0
(22) Date of filing: 08.11.2007
(51) Int. Cl.: G01N 33/74, G01N 33/96, C07K 14/58

(54) **STABLE STANDARDS FOR BNP IMMUNOASSAYS**
STABILE STANDARDS FÜR BNP-IMMUNTESTS
STANDARDS STABLES POUR DES IMMUNODOSAGES DE BNP

(30) Priority: 10.11.2006 US 865397 P; 15.03.2007 FI 20075178
(43) Date of publication of application: 05.08.2009
(73) Proprietor: Hytest Ltd., 20520 Turku (FI)
(72) Inventor: KATRUKHA, Alexei G., F20610 Turku (FI); SEFERYAN, Karina R., Moscow 127427 (RU); TAMM, Natalia N., Moscow 115035 (RU); SEMENOV, Alexander G., Moscow 129128 (RU); POSTNIKOV, Alexander B., Moscow 117192 (RU)
(74) Representative: Karvinen, Leena Maria
(86) International application number: PCT/FI2007/050599
(87) International publication number: WO 2008/056034

(56) References cited:
- WO-A-2007/138163
- WO-A1-2006/027374
- WO-A2-2004/046194
- WO-A2-2006/088624
- WO-A2-2007/047614
- WO-A2-2007/138163
- US-A1- 2004 152 128
- US-A1- 2005 014 287
- US-A1- 2005 148 024
- SHIMIZU HIROYUKI ET AL: "Characterization of molecular forms of probrain natriuretic peptide in human plasma" CLINICA CHIMICA ACTA, ELSEVIER BV, AMSTERDAM, NL, vol. 334, no. 1-2, 1 August 2003 (2003-08-01), pages 233-239, XP002381702 ISSN: 0009-8981
- SCHELLENBERGER UTE ET AL: "The precursor to B-type natriuretic peptide is an O-linked glycoprotein" ARCHIVES OF BIOCHEMISTRY AND BIOPHYSICS, ACADEMIC PRESS, US, vol. 451, no. 2, 15 July 2006 (2006-07-15) , pages 160-166, XP002436954 ISSN: 0003-9861
- GRADDIS T J ET AL: "DISIGNING PROTEINS THAT WORK USING RECOMBINANT TECHNOLOGIES" CURRENT PHARMACEUTICAL BIOTECHNOLOGY, BENTHAM SCIENCE PUBLISHERS, NL, vol. 3, no. 4, 1 December 2002 (2002-12-01), pages 285-297, XP001147744 ISSN: 1389-2010
- TAMM N N ET AL: "Recombinant Proform of Brain Natriuretic Peptide (proBNP), Expressed in Eukaryotic Cells as a Stable Standard fro mBNP Immunoassay" AACC MANUAL MEETING, SAN DIEGO, CA,, 17 July 2007 (2007-07-17), page 1, XP003020785
- SHIMIZU H. ET AL.: 'Characterization of molecular forms of probrain natiruretic peptide in human plasma' CLINICA CHIMICA ACTA vol. 334, 2003, pages 233 - 239, XP002381702
- GRADDIS T.J. ET AL.: 'Designing Proteins That Work Using Recombinant Technologies' CURRENT PHARMACEUTICAL BIOTECHNOLOGY vol. 3, 2002, pages 285 - 297, XP001147744
- SCHELLENBERGER U. ET AL.: 'The precursor to B-type natriuretic peptide is an O-linked glycoprotein' ARCHIVES OF BIOCHEMISTRY AND BIOPHYSICS 2006, pages 160 - 166, XP002436954
- SOLDIN S.J. ET AL.: 'Pediatric brain natriuretic peptide and N-terminal pro-brain natriuretic peptide reference intervals' CLINICA CHIMICA ACTA vol. 366, 2006, pages 304 - 308, XP005320423
- TAMM N.N. ET AL.: 'Recombinant Proform of Brain Natriuretic Peptide (proBNP), Expressed in Eukaryotic Cells as a Stable Standard fro mBNP Immunoassay' AACC MANUAL MEETING, SAN DIEGO, CA 17 July 2007, XP003020785

### SEQUENCE LISTING

<110> Hytest Ltd
<120> Stable standards for BNP immunoassay
<130> 45868
<160> 4
<170> PatentIn version 3.3
<210> 1
   <211> 108
   <212> PRT
   <213> Homo sapiens
<220>
   <221> MISC_FEATURE
   <223> proBNP
<400> 1
   His Pro Leu Gly Ser Pro Gly Ser Ala Ser Asp Leu Glu Thr Ser Gly
   1               5                   10                  15
   Leu Gln Glu Gln Arg Asn His Leu Gln Gly Lys Leu Ser Glu Leu Gln
               20                  25                  30
   Val Glu Gln Thr Ser Leu Glu Pro Leu Gln Glu Ser Pro Arg Pro Thr
           35                  40                  45
   Gly Val Trp Lys Ser Arg Glu Val Ala Thr Glu Gly Ile Arg Gly His
       50                  55                  60
   Arg Lys Met Val Leu Tyr Thr Leu Arg Ala Pro Arg Ser Pro Lys Met
   65                  70                  75                  80
   Val Gln Gly Ser Gly Cys Phe Gly Arg Lys Met Asp Arg Ile Ser Ser
                   85                  90                  95
   Ser Ser Gly Leu Gly Cys Lys Val Leu Arg Arg His
               100                 105
<210> 2
   <211> 134
   <212> PRT
   <213> Homo sapiens
<220>
   <221> MISC_FEATURE
   <223> preproBNP
<400> 2
   Met Asp Pro Gln Thr Ala Pro Ser Arg Ala Leu Leu Leu Leu Leu Phe
   1               5                   10                  15
   Leu His Leu Ala Phe Leu Gly Gly Arg Ser His Pro Leu Gly Ser Pro
               20                  25                  30
   Gly Ser Ala Ser Asp Leu Glu Thr Ser Gly Leu Gln Glu Gln Arg Asn
           35                  40                  45
   His Leu Gln Gly Lys Leu Ser Glu Leu Gln Val Glu Gln Thr Ser Leu
       50                  55                  60
   Glu Pro Leu Gln Glu Ser Pro Arg Pro Thr Gly Val Trp Lys Ser Arg
   65                  70                  75                  80
   Glu Val Ala Thr Glu Gly Ile Arg Gly His Arg Lys Met Val Leu Tyr
                   85                  90                  95
   Thr Leu Arg Ala Pro Arg Ser Pro Lys Met Val Gln Gly Ser Gly Cys
               100                 105                 110
   Phe Gly Arg Lys Met Asp Arg Ile Ser Ser Ser Ser Gly Leu Gly Cys
           115                 120                 125
   Lys Val Leu Arg Arg His
       130
<210> 3
   <211> 76
   <212> PRT
   <213> Homo sapiens
<220>
   <221> MISC_FEATURE
   <223> NT-proBNP
<400> 3
   His Pro Leu Gly Ser Pro Gly Ser Ala Ser Asp Leu Glu Thr Ser Gly
   1               5                   10                  15
   Leu Gln Glu Gln Arg Asn His Leu Gln Gly Lys Leu Ser Glu Leu Gln
               20                  25                  30
   Val Glu Gln Thr Ser Leu Glu Pro Leu Gln Glu Ser Pro Arg Pro Thr
           35                  40                  45
   Gly Val Trp Lys Ser Arg Glu Val Ala Thr Glu Gly Ile Arg Gly His
       50                  55                  60
   Arg Lys Met Val Leu Tyr Thr Leu Arg Ala Pro Arg
   65                  70                  75
<210> 4
   <211> 32
   <212> PRT
   <213> Homo sapiens
<220>
   <221> MISC_FEATURE
   <223> BNP
<400> 4
   Ser Pro Lys Met Val Gln Gly Ser Gly Cys Phe Gly Arg Lys Met Asp
   1               5                   10                  15
   Arg Ile Ser Ser Ser Ser Gly Leu Gly Cys Lys Val Leu Arg Arg His
               20                  25                  30

## Description

### Field of the Invention

The present invention relates to BNP antigen, and particularly to use of a new stable form of said antigen as a standard or calibrator in immunoassays measuring BNP immunoreactivity.

### Background of the Invention

Brain natriuretic peptide (BNP) is a small peptide hormone consisting of 32 amino acid residues. BNP is released from the heart and can be detected in blood by immunological methods. Measurement of the blood concentration of BNP has been recognized as a useful method of identifying patients with congestive heart failure (CHF). A synthetic form of BNP, i.e., BNP-32, is commonly used in immunoassays as a standard. Multiple studies demonstrated significant instability of synthetic BNP-32 when spiked into plasma or buffer solutions. Peptide instability significantly compromises utilization of synthetic BNP-32 in immunoassays as a liquid calibrator or liquid standard.

Brain natriuretic peptide (BNP) is a cardiac neurohormone that is derived from the precursor pre-proBNP, which contains 134 amino acids and includes a signal peptide of 26 amino acids at its N-terminus. proBNP, produced by cleavage of the signal peptide, is further cleaved into BNP, the C-terminal 32 amino acids that is considered to be the biologically active hormone, and what is conventionally believed to be an inactive amino terminal fragment.

Blood measurements of Brain natriuretic peptide (BNP) have been used as diagnostic and prognostic aids in congestive heart failure (CHF), and as a prognostic marker in acute coronary syndromes (ACS). In addition, BNP may prove useful as an aid in guiding medical therapy in patients with CHF. The growing interest in the clinical determination of BNP has led to the development of immunoassays that are suitable for fully automated, high-throughput clinical instruments with random access, e.g., the ADVIA Centaur BNP (Bayer Diagnostics) and the AxSYM BNP system (Abbott Laboratories).

The Advia-Centaur BNP assay is a non-isotopic immunometric method performed on an automated multi-analyzer that allows for high-throughput assay formats. The assay is a two-site immunoassay based on a one-step method. Assay results are visualized by direct chemiluminescence. Although the technology reportedly allows for high-throughput assays with improved accuracy, the Advia-Centaur multi-analyzer provides a platform for the performance of immunoassays. As such, it does not provide improvements to the binding agents used in the assay, such as anti-BNP antibodies.

The AxSYM BNP assay is a fully automated microparticle enzyme immunoassay that uses two monoclonal mouse antibodies in a two-step sandwich format. The BNP is captured on anti-BNP-coated latex microparticles with detection via alkaline phosphatase and measurement of fluorescence produced from the breakdown of 4-methylumbelliferyl phosphate substrate. The solid-phase antibody is directed at the N-terminus of BNP, whereas the conjugate antibody is directed at the C-terminus. Six calibrators are used for the AxSYM BNP assay. Calibrator A is an acetate buffer with protein stabilizers. Calibrators B-F contain increasing concentrations of synthetic BNP (100, 400, 1000, 2000, and 4000 ng/L) in acetate buffer with protein stabilizers. These calibrators are traceable to a reference standard that was prepared gravimetrically with synthetic BNP. The AxSYM BNP assay has a dynamic range of 0-4000 ng/L.

US published patent application No. 2005/014287 discloses stable calibrators and controls for immunoassays of natriuretic peptides involving labeled antibodies. The calibrators and controls described therein are synthetic natriuretic peptides, e.g. ANP or BNP, which are stabilized by adjusting their pH.

The AxSYM BNP assay, like the Advia-Centaur system and other approaches for assaying BNP, use conventional anti-BNP antibodies that specifically recognize an epitope in the mature 32-amino acid BNP. Thus, none of these approaches avoids the constraints on assay accuracy and reproducibility that are imposed by immunological measurement standardization that relies on detection of the mature form of BNP and a need exists in the art for a stable, reliable calibrator or standard for use in measuring BNP.

### Summary of the Invention

Our recent studies have demonstrated that the major part of BNP immunoreactivity detected by immunological methods in human blood is found in the pro-form of BNP (proBNP). Also, recent studies reveal that the major part of proBNP in human blood is glycosylated. These findings establish the benefits of using proBNP as a standard (calibrator) in immunoassays. Stability studies revealed that recombinant proBNP (glycosylated or non-glycosylated) demonstrated significantly higher stability (at least 10- to 1000-fold and even more) being measured in BNP assays in comparison with synthetic BNP-32. The stability of recombinant glycosylated proBNP was significantly higher than the stability of a recombinant protein which was not glycosylated. Consequently, proBNP is superior to synthetic BNP-32 for use in immunoassays as a stable calibrator or standard.

Accordingly, the present invention is directed to use of a peptide selected from the group consisting of an isolated or recombinant or synthetic proBNP consisting of the amino acid sequence given in SEQ ID NO: 1 or a sequence that differs by five or fewer amino acid substitutions, insertions or deletions as a standard or calibrator in a method for detecting BNP immunoreactivity in a sample.

In one embodiment the peptide is an endogenous proBNP isolated from human tissue or body fluids. In a preferred embodiment the proBNP is a glycosylated proBNP.

The glycosylated proBNP may be expressed in eukaryotic cells, preferably in human cells.

A polypeptide having a sequence that differs from SEQ ID NO: 1 by the substitution of five or fewer amino acids (preferably, conservative amino acid substitutions), the insertion of five or fewer amino acids or the deletion of five or fewer amino acids is thus a modification of proBNP.

The amino acid sequence of proBNP is provided in SEQ ID NO: 1.

Other features and advantages of the invention will be better understood by reference to the brief description of the drawing and the detailed description that follow.

### Brief Description of the Drawings

**Figure 1** provides a schematic of preproBNP processing *in vivo.*

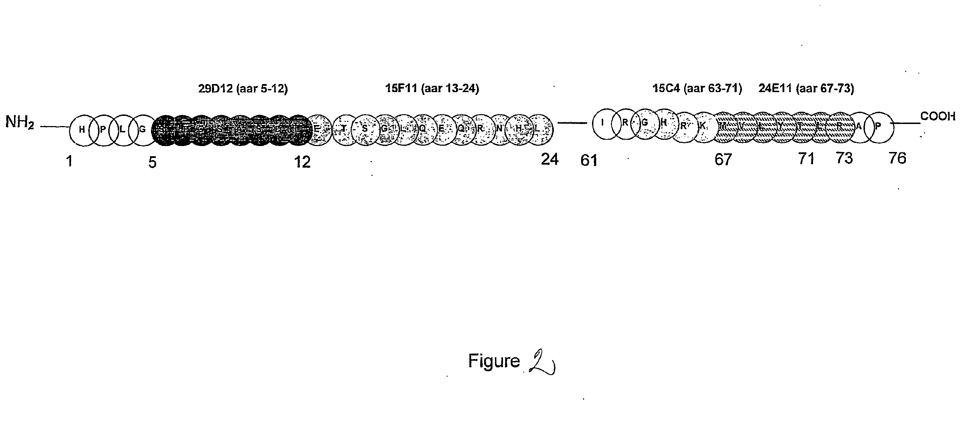

**Figure 2** schematically illustrates epitope locations of monoclonal antibody binding sites suitable for use in, e.g., sandwich immunoassays of BNP immunoreactivity.
**Figure 3** provides a calibration curve for a proBNP sandwich immunoassay. Capture antibody was Mab 50E1 (BNP specific; Hytest Ltd.), detection antibody was MAb 16F3 (NT-proBNP specific; Hytest Ltd.). A one-step assay was performed in streptavidin-coated plates. Biotinylated monoclonal antibodies for capture and Eu-labeled monoclonal antibodies for detection were used. Sample volume: 50 µl, Antigen: human recombinant proBNP, Incubation time: 30 minutes at room temperature.
**Figure 4** is a gel electrophoretograph of Tricine-SDS-PAGE fractionating a sample of recombinant proBNP under reducing conditions. Lane 1: Molecular weight standards (BioRad); Lane 2: rec proBNP (recombinant), 3 µg; Lane 3: rec NT-proBNP (recombinant), 3 µg. The gel was stained with Coomassie brilliant blue R-250.

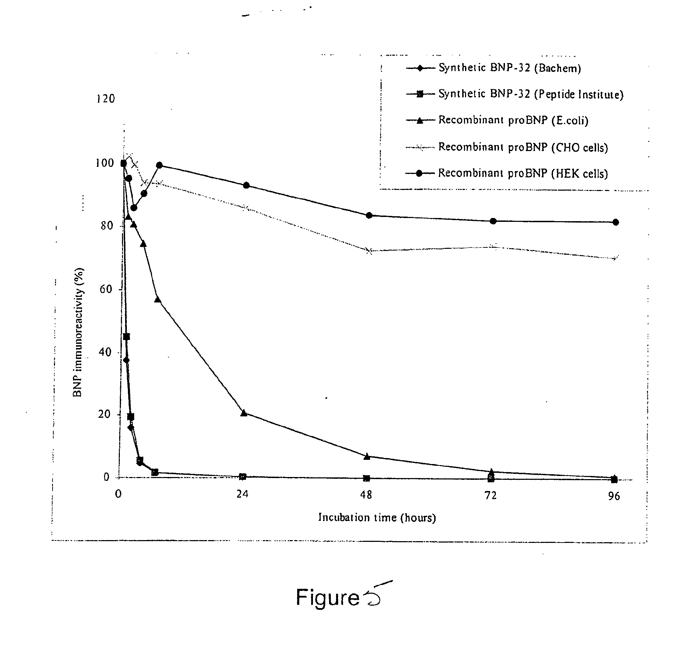

**Figure 5** shows stabilities of different antigens in BNP immunoreactivity measurement. Incubation at 4°C.
**Figure 6** shows stabilities of different antigens in BNP immunoreactivity measurement. Incubation at 25°C.

### Detailed Description of the Invention

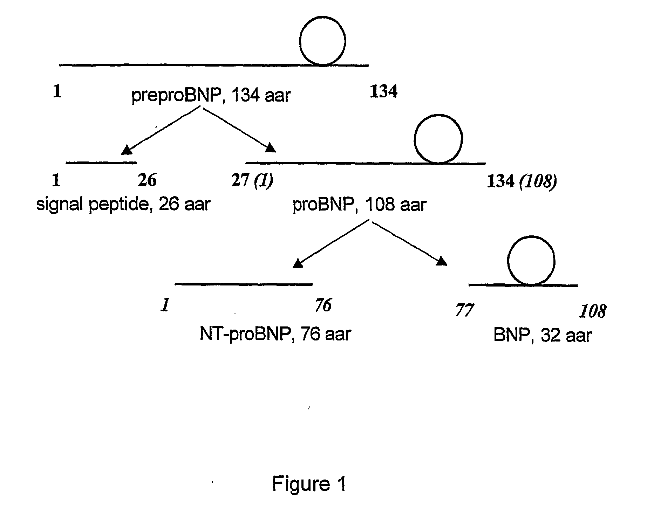

Brain natriuretic peptide (BNP) and NT-proBNP are recognized markers for the diagnosis, prognosis, and treatment of patients with heart failure. Both peptides are products of proteolytic processing of the precursor molecule preproBNP (Fig. 1). PreproBNP is composed of 134 amino acid residues (a.a.r.) and is synthesized in cardiac myocytes. PreproBNP is cleaved by an unknown protease or proteases resulting in two peptides, i.e., a signal peptide (a.a.r. 1-26) and proBNP (a.a.r. 27-134). Further processing of the proBNP molecule (108 a.a.r.) results in the appearance of BNP (a.a.r. 77-108) and the N-terminal part of proBNP, i.e., NT-proBNP, a.a.r. 1-76. The BNP molecule contains a cyclic structure formed by intrinsic disulfide bond formation between two Cys residues. Both BNP (the biologically active molecule) and NT-proBNP (the physiological activity, if any, is not clearly understood) are secreted into the bloodstream in equimolar amounts and circulate in human blood. The BNP concentration in the blood of healthy adults is about 25 pg/ml (Wu et al., Clin Chem. 2004 May; 50(5): 867-73), whereas the NT-proBNP concentration is about 200 pg/ml (Luchner et al., Hypertension, 2002 Jan; 39(1):99-104.). It was established that proBNP synthesis increases in response to mechanical or neurohormonal stimulation of the heart and this increase led to increases of BNP and NT-proBNP concentrations in serum. Elevated levels of BNP and NT-proBNP in human blood are reported for patients with different cardiovascular pathologies, such as heart failure, left ventricular dysfunction, unstable angina and myocardial infarction. Blood concentrations of both analytes in heart failure patients correlate with the severity of disease. It has been reported that both concentrations are already elevated in asymptomatic patients during the very early stage of heart failure (NYHA I stage according to the New York Heart Association classification). Therefore, BNP and NT-proBNP measurements have received interest as a basis for a method for evaluating patients with suspected heart failure. Early diagnosis of heart failure is important for early drug intervention and might diminish the morbidity and mortality associated with advanced stages of these diseases.

BNP and NT-proBNP measurements are useful for risk stratification of patients with different cardiac pathologies. It was demonstrated that patients with possible complications characteristically exhibited significantly higher BNP and NT-proBNP concentrations than patients without complications (Luchner *et al., supra*)*.* For instance, in patients with acute coronary syndrome, measurements of both peptides helped to discriminate those who were at risk of developing new cardiac events. NT-proBNP measurements were helpful not only for disease diagnosis but also for monitoring the therapy administered to patients with heart failure (Troughton et al., Lancet, 2000 Apr 1, 355(9210):1126-30).

At present both analytes are used in clinical practice and there is no agreement between clinicians as to which one is preferable. There is a correlation between NT-proBNP and BNP concentrations and apparently the diagnostic and prognostic values are similar. The NT-proBNP concentration in the blood of patients, however, is 2-5 times higher than the BNP concentration. This fact is explained by the longer half-life of NT-proBNP in patients' blood in comparison with BNP. Thus, the NT-proBNP immunoassay reveals higher sensitivity because of the rather high signal-to-noise ratio characteristic of NT-proBNP assays.

### ProBNP quantitative sandwich immunoassays

BNP precursor (proBNP) is a polypeptide molecule containing 108 amino acid residues. ProBNP can be detected in patients' blood at concentrations comparable with the concentration of circulating BNP and NT-proBNP. Thus proBNP measurements could be of clinical value in cardiovascular disease diagnosis, prognosis, and treatment monitoring. Mabs specific to peptides 5-12 and 13-27 of SEQ ID NO:1 (proBNP) recognize circulating proBNP and could be used for the development of quantitative proBNP assays in pairs with BNP- specific Mabs (e.g., Hytest Ltd. cat. no. 4BNP2). Preferred Mab pairs (capture - detection) are:
50E1 - 16F3
50E1 - 18H5
7B5 - 2G9

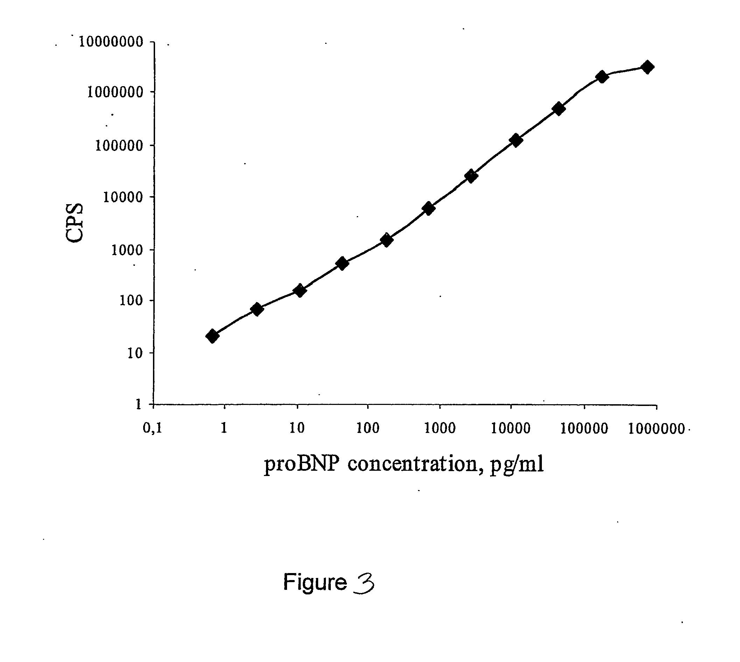

Each of these pairs of anti-proBNP monoclonal antibodies demonstrates high sensitivity, good kinetics and effective recognition of antigen in patients' blood (Fig. 3).

### NT-proBNP and proBNP immunodetection by Western blotting

Hytest Ltd. produces monoclonal antibodies which recognize human NT-proBNP and proBNP and may be used in Western blotting studies after antigen transfer onto nitrocellulose membrane.

The human recombinant NT-proBNP and proBNP used in the experiments described herein exhibit the following properties:

| | |
|---|---|
| Purity: | > 98 % according to SDS-PAGE |
| Application: | NT-proBNP and proBNP immunoassay calibrators and standards, immunoblotting. |

Human recombinant NT-proBNP (rec NT-proBNP; a.a.r. 1-76; SEQ ID NO:3) and human recombinant proBNP (rec proBNP; a.a.r. 1-108; SEQ ID NO:1) are expressed in E. *coli.* Both polypeptides have the same sequence as natural proteins with a single amino acid difference, an additional Met residue from the N-terminus of the molecule. Antigens are recognized by poly- and monoclonal antibodies specific to different parts of NT-proBNP. ProBNP is also recognized by antibodies specific to BNP.

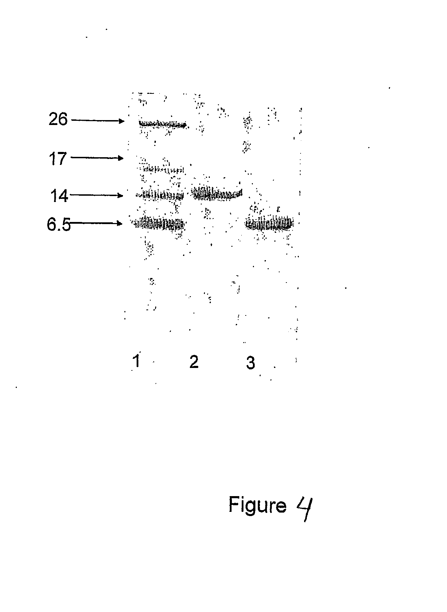

Both rec NT-proBNP and rec proBNP (Hytest Ltd.) are highly purified peptides. Purity is more than 98 % according to SDS-PAGE (Fig. 4) and HPLC studies. The antigens could be used as calibrators or as standards in NT-proBNP and proBNP assays. In BNP clinical immunoassays, proBNP is preferred as a standard or calibrator. Also useful as a calibrator in BNP immunoassays is a modified form of proBNP, as defined hereinabove.

Although a variety of BNP immunoassays are useful in measuring BNP immunoreactivity in a variety of samples, superior results are obtained from immunoassays in which a stable standard or calibrator peptide, such as proBNP, is used. The stability studies described below show that recombinant proBNP demonstrated significantly higher stability than synthetic BNP-32. In addition, among recombinant proBNPs, the glycosylated forms of proBNPs expressed in eukaryotic cell lines are significantly more stable than non-glycosylated proBNPs expressed in bacterial cells.

### Example 1

### Stability studies.

**Antigens used in the studies.** The antigens used in the stability studies included synthetic BNP-32 (Bachem, Bubendorf, Switzerland, cat. no. H-9060.0500), synthetic BNP-32 (Peptide Institute, Osaka, Japan, cat. no. 4212-v), recombinant proBNP (expressed in *E. coli,* non-glycosylated, from Hytest Ltd., Turku, Finland, 8PRO8), recombinant proBNP (expressed in eukaryotic CHO (Chinese hamster) cells and, thus, glycosylated, as described herein), and recombinant proBNP (expressed in eukaryotic HEK (human) cells and, thus, glycosylated, as described herein).

**Antigen solutions.** An antigen matrix was prepared by pooling human citrate plasmas collected from healthy donors. Plasma was obtained from 15 healthy volunteers by vein puncture and mixed with 0.11 M sodium citrate tribasic in a ratio of 4:1. The crude serum:citrate was centrifuged for 30 minutes at 2000 rpm. Plasma was decanted, pooled and stored at -70°C until use. Antigen concentrations used in the studies were 6 ng/ml for recombinant proBNP expressed in CHO cells, 20 ng/ml for synthetic BNPs, 20 ng/ml for recombinant proBNP expressed in *E. coli* and 20 ng/ml for recombinant proBNP expressed in HEK cells. 0.1 % sodium azide was added to plasma as a preservative to prevent bacterial growth.

**Incubation at 4°C and 25°C.** Antigen solutions in human plasma were incubated for different time periods (up to 96 hours) at 4°C and 25°C. Aliquots were frozen and stored at -70°C until BNP immunoreactivity was measured.

**Assays for BNP immunoreactivity. BNP** immunoreactivity in the samples was determined by sandwich immunofluorometric assay utilizing two BNP-specific monoclonal antibodies (MAbs). A first BNP-specific monoclonal antibody was coating monoclonal antibody, or MAb, 50E1 (Hytest Ltd. cat, no. 4BNP2), which specifically recognized and bound to peptide 26-32 of the BNP sequence. A second BNP-specific monoclonal antibody was detection monoclonal antibody, or MAb, 24C5 (Hytest Ltd. cat. no. 4BNP2). Also suitable for use in such assays are a variety of antibodies specific to BNP, such as additional antibodies provided by Hytest Ltd. under cat. no. 4BNP2, i.e., 26E2, 2G9, 50B7, 57H3, 41A6, 43B12, and 16B 12, as well as other monoclonal or polyclonal antibodies of various isotypes, and fragments or derivatives that retain the capacity to specifically bind to BNP.

MAb 24C5 specifically recognized and bound to peptide 11-22 of the BNP sequence and was conjugated to stable Eu³⁺ chelate using conventional techniques. Such an assay is able to detect not only BNP but also proBNP. Consequently, it can be used for the quantification of whole BNP immunoreactivity, comprising both BNP and proBNP.

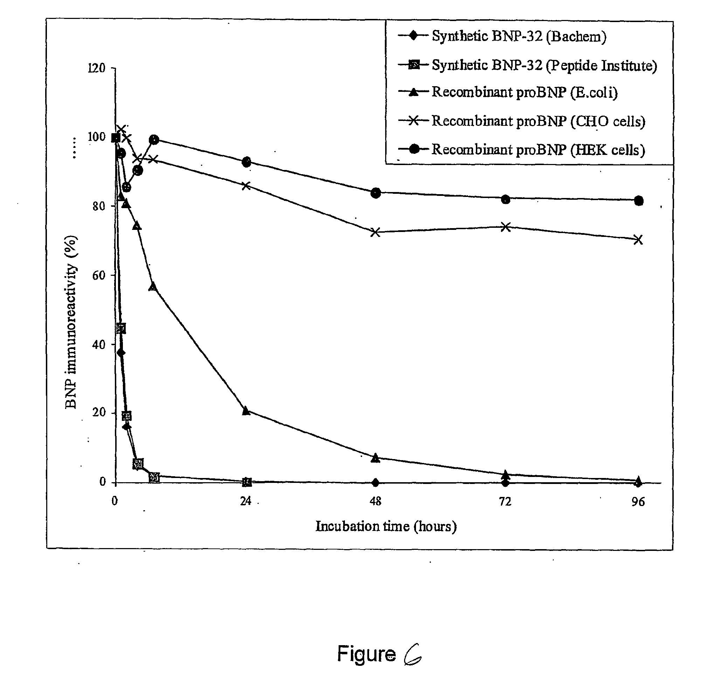

The results of the stability studies are presented in Fig. 6 and Fig. 7. Synthetic BNP-32 demonstrated the worst stability among all of the tested protein forms displaying BNP immunoreactivity. 3% and less of initial immunoreactivity were observed in the samples after 24 hours of incubation at 4°C and less than 0.5% after 24 hours of incubation at 25°C. Almost no immunoreactivity was detected after 96 hours of incubation at both temperatures.

Recombinant non-glycosylated proBNP, expressed in *E*. *coli,* demonstrated intermediate stability. More than 50% of initial BNP immunoreactivity was still detected after 96 hours of incubation at 4°C. At 25°C recombinant proBNP, expressed in *E*. *coli,* demonstrated significantly lower stability than after incubation at 4°C, i.e., less than 1% of initial BNP immunoreactivity was detected after 96 hours of incubation at 25°C.

Recombinant glycosylated forms of proBNP expressed in CHO or HEK cells demonstrated the highest stabilities, with higher stability for the proBNP expressed in HEK cells. More than 90% of the initial immunoreactivity was observed for both proteins after 96 hours of incubation at 4°C; 70% and 82% of initial immunoreactivity for proteins expressed in CHO and HEK cell lines, respectively, were observed after 96 hours of incubation at 25°C. At both temperatures, the stability of the antigen expressed in human HEK cells during long-term incubation was higher than the stability of the antigen expressed in CHO cells.

In conclusion, recombinant proBNPs demonstrated significantly increased stability of BNP immunoreactivity in comparison with synthetic BNP-32 molecules. Further, glycosylated recombinant proBNPs expressed in eukaryotic cell lines were significantly more stable than non-glycosylated proBNP expressed in *E*. *coli.*

The data disclosed herein, along with the additional disclosure, establishes that immunoassays of proBNP forms of Brain Natriuretic Peptide, also modified forms of proBNP, provide materials for preparations and methods for standardizing such assays in a manner that results in improved accuracy and reproducibility of the assay results. These improvements, in turn, lead to more accurate and reliable diagnoses of cardiovascular disease, such as congestive heart failure. In addition, the improved assays are useful for prognostic applications and the monitoring of treatment for patients exhibiting, or at risk of exhibiting, cardiovascular disease or symptoms characteristic thereof.

## Claims

1. Use of a peptide selected from the group consisting of an isolated or recombinant or synthetic proBNP consisting of the amino acid sequence given in SEQ ID NO:1 or a sequence that differs by five or fewer amino acid substitutions, insertions or deletions as a standard or calibrator in a method for detecting BNP immunoreactivity in a sample.

2. Use according to claim 1, wherein the peptide is an endogenous proBNP isolated from human tissue or body fluids.

3. Use according to claim 1, wherein the proBNP is a glycosylated proBNP.

4. Use according to claim 3, wherein the glycosylated proBNP is expressed in eukaryotic cells.

5. Use according to claim 4, wherein the glycosylated proBNP is expressed in human cells.

## Patentansprüche

1. Verwendung eines Peptids, ausgewählt aus der Gruppe bestehend aus einem isolierten oder rekombinanten oder synthetischen ProBNP, das aus der in SEQ ID NO: 1 angegebenen Aminosäuresequenz oder einer Sequenz besteht, die sich durch fünf oder weniger Aminosäuresubstitutionen, -insertionen oder -deletionen davon unterscheidet, als Standard oder Kalibrator in einem Verfahren zur Bestimmung der BNP-Immunoreaktivität in einer Probe.

2. Verwendung nach Anspruch 1, wobei das Peptid ein endogenes ProBNP, isoliert aus menschlichem Gewebe oder Körperflüssigkeiten, ist.

3. Verwendung nach Anspruch 1, wobei das ProBNP glykosyliertes ProBNP ist.

4. Verwendung nach Anspruch 3, wobei das glykosylierte ProBNP in eukaryotischen Zellen exprimiert wird.

5. Verwendung nach Anspruch 4, wobei das glykosylierte ProBNP in menschlichen Zellen exprimiert wird.

## Revendications

1. Utilisation d'un peptide choisi dans le groupe comprenant un proBNP isolé ou recombinant ou synthétique constitué de la séquence d'acides aminés donnée par SEQ ID NO:1 ou d'une séquence qui diffère par cinq substitutions, insertions ou délétions ou moins d'acides aminés en tant qu'étalon ou calibrateur dans un procédé de détection de l'immunoréactivité du BNP dans un échantillon.

2. Utilisation selon la revendication 1, où le peptide est un proBNP endogène isolé de tissu ou de liquides corporels humains.

3. Utilisation selon la revendication 1, où le proBNP est un proBNP glycosylé.

4. Utilisation selon la revendication 3, où le proBNP glycosylé est exprimé dans des cellules eucaryotes.

5. Utilisation selon la revendication 4, où le proBNP glycosylé est exprimé dans des cellules humaines.
